# EUROPEAN PATENT APPLICATION

(11) **EP 4 772 607 A1**
(43) Date of publication of application: **08.07.2026**
(21) Application number: 24859002.8
(22) Date of filing: 18.03.2024
(51) Int. Cl.: C12N 1/00, C12Q 1/18

(54) **SCREENING METHOD AND SOLUTION**

(30) Priority: 29.08.2023 JP 2023139257
(71) Applicant: Hiroshima University, Higashi-Hiroshima-shi Hiroshima 739-8511 (JP)
(72) Inventor: AOI, Yoshiteru, Hiroshima-shi, Hiroshima 739-8511 (JP); SHIMOMURA, Yumi, Hiroshima-shi, Hiroshima 739-8511 (JP); NIIYAMA, Una, Hiroshima-shi, Hiroshima 739-8511 (JP)
(74) Representative: Plasseraud IP
(86) International application number: PCT/JP2024/010423
(87) International publication number: WO 2025/046952

(57) **Abstract**

Provided is a screening method by which a microorganism that has a desired activity can be selected with high throughput. A screening method for searching for a microorganism (12) that has a desired activity includes: a particle forming step of obtaining a multilayer particle (10) by forming, outside a core particle (11) formed from a solid culture medium containing the microorganism (12), at least one detection layer (13) that is composed of a gel or an aqueous solution and that contains a detection body (14) which detects the desired activity; and a selection step of selecting the multilayer particle (10) on the basis of a signal indicating a result of detection of the desired activity by the detection body (14), in a case where the detection layer (13) is formed as a layer that is composed of the aqueous solution, the layer being formed as an outermost layer of the multilayer particle (10) in a hydrophobic solvent (20).

## Description

### Technical Field

The present invention relates to a microorganism screening method using a multilayer particle, and to a multilayer particle-containing solution.

### Background Art

Development of novel compounds that are useful for drug development, such as novel antibacterial substances, enzymes that enable novel reactions, and unused bioresources such as microorganisms that produce the novel compounds and the enzymes is recognized as an important issue. As an example of such development, it is expected to use, as resources, unused microorganisms that have not been discovered because they are uncultured and that are present in the environment, and microorganisms that have been obtained by modifying properties of existing microorganisms which are already cultured.

However, the proportion of microorganisms having a specific desired activity, among microorganisms present in the environment is ordinarily extremely low. Screening for the purpose of (i) isolating, from the environment, a microorganism that has a desired activity and (ii) modifying or improving properties of cultured microorganisms requires large-scale screening of 1,000,000 sample units.

For example, Non-patent Literature 1 discloses a microorganism culture method using gel microparticles. According to such a culture method, microorganisms different from each other can be cultured in each of a large number of gel microparticles dispersed in a hydrogel.

Patent Literature 1 discloses a method for aggregating hydrogel microparticles in oil. Such a method allows a dissolved substance and ions to pass between the hydrogel microparticles. This makes it possible to strengthen an intercellular interaction while maintaining isolation culture of microorganisms for each hydrogel microparticle, so that culture efficiency is improved.

### Citation List

### [Patent Literature]

[Patent Literature 1]
Japanese Patent Application Publication Tokukai No. 2017-063764

### [Non-patent Literature]

[Non-patent Literature 1]
Karsten Zengler et. al., PNAS, Vol. 99, No. 24, pp. 15681-15686, 2002

### Summary of Invention

### Technical Problem

However, a conventional method using these gel microparticles makes it difficult to add, with high throughput after culturing microorganisms, detection bodies such as a cell and a substrate for detecting a desired activity.

An aspect of the present invention has an object to achieve a screening method by which a microorganism that has a desired activity can be selected with high throughput.

### Solution to Problem

In order to attain the object, a screening method in accordance with an aspect of the present invention is a screening method for searching for a desired microorganism that is included in a group of microorganisms and that has a desired activity, the screening method including: a particle forming step of obtaining a multilayer particle by forming, outside a core particle formed from a solid culture medium containing the microorganisms, at least one detection layer that is composed of a gel or an aqueous solution and that contains a detection body which detects the desired activity; and a selection step of selecting, on the basis of a signal indicating a result of detection of the desired activity by the detection body, the multilayer particle that contains the desired microorganism, in the particle forming step, in a case where the at least one detection layer is formed as a layer that is composed of the aqueous solution, the layer being formed as an outermost layer of the multilayer particle in a hydrophobic solvent.

In order to attain the object, a solution in accordance with an aspect of the present invention is a solution which is used to search for a desired microorganism that is included in a group of microorganisms and that has a desired activity and which contains a multilayer particle and a hydrophobic solvent, the multilayer particle including: a core particle formed from a solid culture medium containing the microorganisms; and at least one detection layer that is formed outside the core particle, that is composed of a gel or an aqueous solution, and that contains a detection body which detects the desired activity, wherein in a case where the multilayer particle includes the at least one detection layer that is composed of the aqueous solution, the at least one detection layer that is composed of the aqueous solution is an outermost layer of the multilayer particle.

### Advantageous Effects of Invention

An aspect of the present invention makes it possible to achieve a screening method by which a microorganism that has a desired activity can be selected with high throughput.

### Brief Description of Drawings

Fig. 1 is a view schematically illustrating a multilayer particle-containing solution in accordance with an embodiment of the present invention.
Fig. 2 is a flowchart showing a screening method in accordance with an embodiment of the present invention.
Fig. 3 is a view of a microscopic image of a core particle that contains lactic acid bacteria.
Fig. 4 is a view of a microscopic image of the core particle illustrated in Fig. 3, the microscopic image having been obtained after culturing.
Fig. 5 is a view of a microscopic image of a multilayer particle that contains lactic acid bacteria and Escherichia coli serving as a detection body.
Fig. 6 is a view of a microscopic image of a multilayer particle that does not contain Escherichia coli serving as a detection body.
Fig. 7 is a view of a microscopic image of the multilayer particle illustrated in Fig. 5, the microscopic image having been obtained after culturing.
Fig. 8 is a view illustrating a dot plot and a set area in a cell sorter for a sample that contains the multilayer particle illustrated in Fig. 6.
Fig. 9 is a view of a microscopic image of a multilayer particle and a core particle that have been sorted from an area B illustrated in Fig. 8.
Fig. 10 is a view illustrating a dot plot and a set area in a cell sorter for a sample that contains the multilayer particle illustrated in Fig. 7.
Fig. 11 is a view of a microscopic image of a multilayer particle that has been sorted from the area B illustrated in Fig. 9.
Fig. 12 is a view of a microscopic image of a multilayer particle that has been sorted from an area C illustrated in Fig. 9.
Fig. 13 is a view of a microscopic image of a core particle that contains cellulase-producing bacteria.
Fig. 14 is a view of a microscopic image of a multilayer particle that has a detection layer which is composed of an aqueous solution.
Fig. 15 is a view of microscopic images of the multilayer particle illustrated in Fig. 14, the microscopic images having been obtained before and after culturing.
Fig. 16 is a view of microscopic images of a multilayer particle that contains soil bacteria in a core particle, the microscopic images having been obtained before and after culturing.

### Description of Embodiments

The following description will discuss an embodiment of the present invention. Note that, in the present specification, a numerical range "A to B" means "not less than A and not more than B".

### [Overview of screening method]

A screening method in accordance with an embodiment of the present invention is a method for searching for a desired microorganism that is included in a group of microorganisms and that has a desired activity.

It is considered that a microorganism which has not been discovered because the microorganism is artificially difficult to culture and which has a useful activity is present in the environment. However, conventionally, there are large bottlenecks in searching for and acquiring a useful microorganism that produces a useful enzyme, compound, or the like. One of the bottlenecks is that the proportion of microorganisms having a specific desired activity, among microorganisms in the environment is extremely low.

That is, screening of a mutant strain for the purpose of (i) isolating one type of microorganism from the environment or (ii) modifying and improving properties of cultured microbial strains requires a scale of 1,000,000 sample units. Further, there are too many types of target microorganisms to analyze specific substance-producing ability of a microorganism (a type and the amount of substance produced, the expression level and expression trend of a gene, and the like). This makes it extremely difficult to use the prior art to efficiently advance an analysis.

Thus, there is a need for a technology for screening, with ultrahigh throughput which is far beyond that in the prior art, a microorganism which has a desired activity. Analysis and selection methods with ultrahigh throughput are required also for breeding selected useful microorganisms, i.e., creating mutant strains to screen a mutant strain that has a further improved function and/or activity.

In contrast, simply increasing the number of measurable samples (sample number) cannot be said to be sufficient. This is because, in order to detect a specific activity of a candidate microorganism, it is necessary to separately add an additive that contains a detection body necessary for detection of the specific activity, and, in addition to this, a high-throughput operation is required.

In particular, it is necessary in many cases to culture microorganisms for a certain period of time for a specific activity of the microorganisms to be expressed. Furthermore, in many cases, one cell is insufficient to detect expression of a specific activity, and a step of detecting the expression after proliferating the same microorganisms is necessary. Moreover, in a case where an additive that contains a detection body necessary for detection cannot be added simultaneously during culture of microorganisms to be detected, for example, the additive adversely affects proliferation and culture of the microorganisms in many cases.

In such a case, the additive that contains the detection body is preferably added after the microorganisms are cultured for a prescribed period. In a case where a container that is commonly used in screening, such as a multiwell microplate is used (100 to 1,000 sample units), an existing device and an existing tool can be used to relatively easily add the additive at a desired timing.

However, in the case of ultrahigh-throughput screening of 1,000,000 or more sample units, the volume of liquid is extremely small, and an operation of adding an additive to a container later is extremely difficult. Such an operation is impossible in principle particularly in a case where a microdroplet with a diameter of 1 mm or less is used. Thus, high-throughput screening using a microdroplet technology is limited in applicable scope and is actually not practically used.

The screening method in accordance with an embodiment of the present invention makes it possible to rapidly and relatively easily detect and select, from a variety of microorganisms in the environment or a large number of mutant strains, a microorganism that has a desired activity (desired microorganism).

In particular, an embodiment of the present invention makes it possible to provide a screening method by which an additive that contains a detection body necessary for detection of a specific activity can be added at a desired timing in a screening system using the microdroplet technology. The screening method is an extremely innovative method in that the screening method makes it possible to carry out high-throughput screening while, utilizing as it is an advantage of the microdroplet technology of making it possible to culture many of microorganisms which have been difficult to culture on a plate medium.

Such a configuration makes it possible to effectively promote use of a useful activity that a microorganism has, that is, effective use of natural resources. Such an effect also contributes to achieving, for example, Goal 12.2 "Sustainable Management and Efficient Use of Natural Resources" of the Sustainable Development Goals (SDGs) proposed by the United Nations.

### [Multilayer particle]

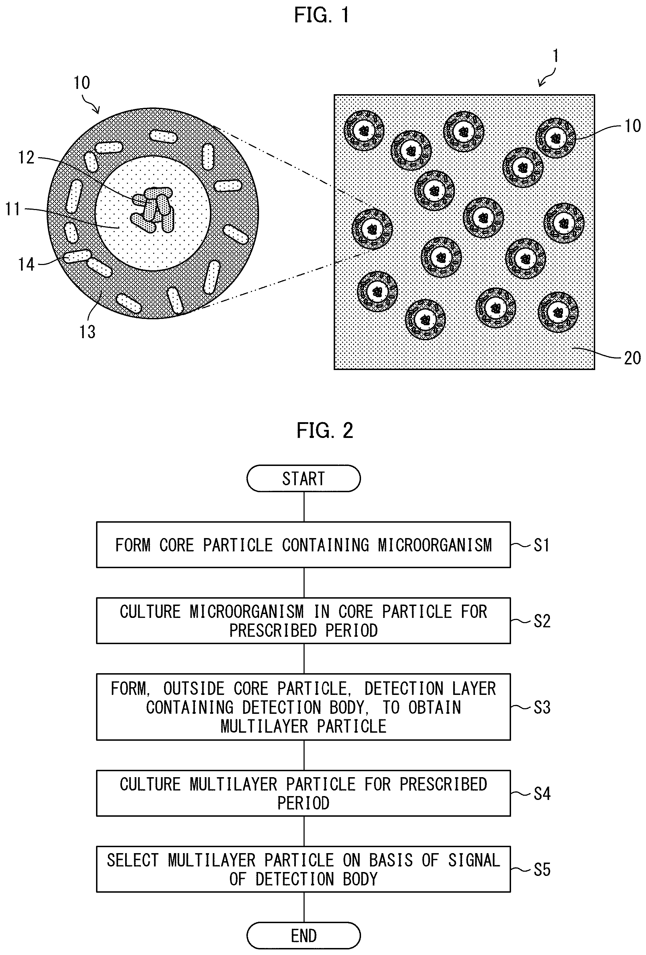

The screening method in accordance with an embodiment of the present invention is carried out with use of a multilayer particle 10 illustrated in Fig. 1. Fig. 1 is a view schematically illustrating an example of a solution 1 containing the multilayer particle 10.

The multilayer particle 10 includes a core particle 11 and a detection layer 13. In the present embodiment, an example in which the multilayer particle 10 includes these two layers is used to provide a description. However, the present embodiment is not limited to this example. The multilayer particle 10 may include three or more layers. For example, the multilayer particle 10 may include a plurality of core particles 11, may include a plurality of detection layers 13, and/or may further include a layer other than the core particle 11 and the detection layer 13.

The multilayer particle 10 is not particularly limited in size. For example, the multilayer particle 10 may have a diameter of 1 µm to 1 mm. In a case where the multilayer particle 10 has a shape different from a spherical shape, a long diameter indicating a distance between two virtual points with the longest distance in the multilayer particle 10 is preferably in the foregoing numerical range of the diameter.

### (Core particle)

The core particle 11 is formed from a solid culture medium containing a microorganism 12. The core particle 11 constitutes an innermost layer of the multilayer particle 10. The core particle 11 is not particularly limited in size. For example, the core particle 11 may have a diameter or long diameter of 1 µm to 1 mm.

The microorganism 12 that is contained in the core particle 11 is not particularly limited but may be a prokaryote or eukaryote, such as bacteria and archaea. The microorganism 12 is preferably a unicellular organism but may be a multicellular organism. The microorganism 12 may be a microorganism that is present in the environment, may be a microorganism that has been artificially cultured, or may be a microorganism that has been further artificially subjected to mutagenesis. In a case where a microorganism that is present in the environment is used as a screening target, the microorganism 12 may be obtained, for example, from any of river water, soil, a biofilm, and the like.

The core particle 11 preferably contains a single microorganism 12 when the core particle 11 is initially formed. The microorganism 12 that is contained in the core particle 11 can be proliferated by culturing the multilayer particle 10. The core particle 11 can contain, for example, 100 to 1,000 microorganisms 12, depending on the size of the core particle 11.

The solid culture medium from which the core particle 11 is formed may be configured as, for example, a hydrogel containing agarose, agar, gelatin, or the like. The solid culture medium may contain a nutrient source that is used to culture the microorganism 12, or may further contain another component such as a buffer.

The nutrient source that is contained in the solid culture medium is not particularly limited. However, in a case where it is possible to estimate a nutrient source required by the microorganism 12 that has a desired activity, the nutrient source that is contained in the solid culture medium preferably also contains the nutrient source required by the microorganism 12. In a case where the nutrient source required by the microorganism 12 that has the desired activity is unclear, the nutrient source that is contained in an individual culture medium may be selected in accordance with a commonly known culture medium composition for a microorganism. Note that, in a case where the microorganism 12 that has the desired activity can be estimated to be culturable if there is no nutrient source but water, the solid culture medium need not contain any nutrient source.

### (Detection layer)

At least one detection layer 13 is formed, outside the core particle 11, as a layer that is composed of a gel or an aqueous solution and that contains a detection body 14 which detects the desired activity.

As in the case of the core particle 11, the detection layer 13 may be configured as a hydrogel containing agarose, agar, gelatin, or the like. Further, as in the case of the core particle 11, the detection layer 13 may further contain a component such as a nutrient source.

The detection body 14 functions as a sensor for detecting the microorganism 12 that has the desired activity. The desired activity may be an activity of a substance secreted by the microorganism 12, or may be an activity exhibited by the microorganism 12 itself. The activity of the substance secreted by the microorganism 12 may be, for example, antimicrobial activity that is caused by an antibacterial substance, may be cellulase activity that cleaves a glycoside bond which cellulose or the like has, or may be physiological activity of a substance that has other physiological activity. The activity of the substance secreted by the microorganism 12 may be, for example, catalytic activity for a specific reaction by an enzyme or the like secreted by the microorganism 12. Examples of the activity exhibited by the microorganism 12 itself include production activity of an industrially useful substance by the microorganism 12.

The detection body 14 may be selected as appropriate in accordance with the desired activity. For example, in a case where the desired activity is antimicrobial activity, the detection body 14 may be a cell different from the microorganism 12 that has the desired activity. Examples of the cell different from the microorganism 12 that has the desired activity include a cell that is assumed to have sensitivity to antimicrobial activity of the microorganism 12, e.g., a microorganism and an animal cell. In a case where the detection body 14 is such a cell, detecting whether the cell is alive or dead makes it possible to select the microorganism 12 that has the desired activity.

In a case where the desired activity is antimicrobial activity that is also effective against existing drug-resistant bacteria, the detection body 14 may be known drug-resistant bacteria. The drug-resistant bacteria may be drug-resistant bacteria against a single antibacterial substance, and is more preferably multi-drug resistant bacteria in terms of finding a novel antibacterial substance. Such a configuration makes it possible to screen the microorganism 12 that also exhibits effective antimicrobial activity against existing drug-resistant bacteria.

In a case where the detection body 14 is a cell, the cell is preferably a cell into which a fluorescent substance or a light-emitting substance has been introduced. In this case, a reduction in fluorescence emitted by the fluorescent substance or in light emission by the light-emitting substance, the reduction being caused by a reduction in number of cells, each of which is the detection body 14, can be used as a signal which indicates that the detection body 14 has detected the desired activity. Further, the cell that serves as the detection body 14 may be a cell such that the desired activity of the microorganism 12 promotes or suppresses production of the introduced fluorescent substance or light-emitting substance.

According to such a configuration, in a case where cell survival or death or the presence or absence of cell proliferation is used as an indicator of the desired activity, the desired activity can be easily detected by using, as a signal, fluorescence emitted by a fluorescent substance or light emission by a light-emitting substance. The cell into which a fluorescent substance or a light-emitting substance has been introduced may be a cell into which a gene that expresses, for example, a fluorescent protein or a light-emitting protein has been introduced. A light-emitting protein may be, for example, a light-emitting enzyme or an enzyme that catalyzes conversion of a light-emitting substance precursor into a light-emitting substance.

Further, cell survival or death or the presence or absence of cell proliferation may be detected with use of a reagent or the like that can detect a living cell or a dead cell. Thus, the detection body 14 need only be a substance that receives some influence from the desired activity that the microorganism 12 has, and need not spontaneously emit a signal that visualizes the influence. A signal that visualizes whether the detection body 14 has received the influence may be indirectly generated with use of a reagent or the like.

Such a reagent or the like for visualizing a result of detection by the detection body 14 may be added to a solution into which the multilayer particle 10 disperses, or another layer containing the reagent or the like may be further formed in the multilayer particle 10. Further, the detection layer 13 may also contain, in addition to the detection body 14, a substance for visualizing the influence that the detection body 14 has received.

In a case where the detection body 14 is a cell, the detection layer 13 preferably contains, for example, a nutrient source that is used for proliferation of the cell. In a case where a cell that serves as the detection body 14 is capable of proliferating in the detection layer 13, the presence or absence of proliferation of the cell can be used as an indicator of the desired activity.

In a case where the desired activity is a specific enzyme activity, the detection body 14 may be, for example, a substrate of an enzyme. In a case where the detection body 14 is a substrate of an enzyme, the substrate is preferably labeled with a fluorescent substance, a light-emitting substance, or the like, but is not limited to this. For example, a substrate that is labeled with a fluorescent substance, a light-emitting substance, or the like does not emit fluorescence or light as it is, but may be converted into a fluorescence or light-emitting substance by the desired activity of the microorganism 12. Such a desired activity of the microorganism 12 may be, for example, enzyme activity that, for example, degrades the substrate. For example, in a case where the detection body 14 is an unlabeled substrate, an influence on the unlabeled substrate by an enzyme may be detected with use of a reagent or the like.

In a case where the desired activity is a specific enzyme activity, the specific enzyme activity is not particularly limited but may include, for example, industrially used enzymes such as amylase, cellulase, protease, lipase, glycosidase, and transferase.

In a case where the desired activity is cellulase activity, the detection body 14 may be a fluorescent substance that emits fluorescence due to an influence of the cellulase activity or a light-emitting substance that emits light due to the influence of the cellulase activity. The aforementioned fluorescent substance may be a fluorescent substrate that has a glycoside bond serving as a substrate of cellulase and that emits fluorescence upon cleavage of the glycoside bond.

Examples of such a fluorescent substrate include a fluorescent substance-cellulose-bonded compound such as fluorescein di-beta-D-cellobioside. The aforementioned light-emitting substance may be a light-emitting substrate that has a glycoside bond serving as a substrate of cellulase and that emits light upon cleavage of the glycoside bond. Examples of such a light-emitting substrate include a cellulose-light-emitting substance-bonded compound such as resorufin β-D-cellobioside.

According to such a fluorescent substance or light-emitting substance, in selection of the microorganism 12 that has the cellulase activity, an increase in fluorescence emitted by the fluorescent substance or in light emission by the light-emitting substance can be used as the signal which indicates that the detection body 14 has detected the desired activity.

The detection layer 13 has a thickness that is not particularly limited and may be, for example, 1 µm to 1 mm. The detection layer 13 is preferably formed so as to have a thickness that is substantially uniform. In a case where the detection layer 13 have different thicknesses depending on its position in the multilayer particle 10, an average of the thicknesses is preferably in the aforementioned numerical range.

Two or more detection layers 13 may be formed outside the core particle 11. In this case, the detection layers 13 may be layers that contain different types of the detection body 14. For example, in a case where a plurality of desired activities are set in terms of, for example, an increase in efficiency, a case is assumed where a period during which the microorganism 12 should be cultured differs for each of the desired activities. In this case, one detection layer 13 may be formed first to detect a corresponding desired activity. For the multilayer particle 10 that has not been selected, culture may be further advanced, and another detection layer 13 may be further formed to detect a corresponding desired activity again.

The multilayer particle 10 may include a single core particle 11 or a plurality of core particles 11 inside the detection layer 13. In a case where the multilayer particle 10 includes a single core particle 11, the microorganism 12 that has the desired activity can be selected with higher accuracy. In a case where the multilayer particle 10 includes a plurality of core particles 11, the microorganism 12 that has the desired activity can be selected with higher throughput.

The detection layer 13 may be a layer that is composed of an aqueous solution rather than a gel. In a case where the multilayer particle 10 includes the detection layer 13 that is composed of an aqueous solution, the detection layer 13 that is composed of the aqueous solution is formed as an outermost layer of the multilayer particle 10. Such a multilayer particle 10 can be formed by being dispersed in a hydrophobic solvent 20. By using the outermost layer of the multilayer particle 10 as the detection layer 13 that is composed of the aqueous solution, it is possible to, for example, easily adjust a particle size of the multilayer particle 10.

In a case where the detection layer 13 which is the outermost layer of the multilayer particle 10 is composed of the aqueous solution, the detection body 14 that is contained in the detection layer 13 may be a water-soluble compound. Such a multilayer particle 10 is dispersed in the hydrophobic solvent 20 so as to be maintained. Thus, the detection body 14 that is the water-soluble compound almost does not leak from the detection layer 13 into the hydrophobic solvent 20.

In contrast, in a case where the detection layer 13 which is the outermost layer of the multilayer particle 10 is composed of a gel, the multilayer particle 10 can be dispersed in water or an aqueous solution. In this case, in a case where the detection body 14 that is contained in the outermost detection layer 13 is the water-soluble compound, the detection body 14 can leak from the detection layer 13 into the water or aqueous solution depending on the size of the detection body 14. Such an event is highly likely to occur in a case where the detection body 14 has a smaller size than, for example, a molecular network of the gel in the detection layer 13.

Thus, in a case where the detection body 14 is the water-soluble compound, the multilayer particle 10 preferably continues to be dispersed in the hydrophobic solvent in order to favorably detect the signal of the detection body 14. In such a case, the detection layer which is the outermost layer of the multilayer particle 10 can also be composed of an aqueous solution rather than a gel. Examples of the detection body 14 that is the water-soluble compound include a substrate of an enzyme. Such a substrate of an enzyme can be exemplified by a fluorescent substance that emits fluorescence due to an influence of the cellulase activity and a light-emitting substance that emits light due to the influence of the cellulase activity, as described earlier.

### (Solution)

The solution 1 is used to search for a desired microorganism that is included in a group of microorganisms 12 and that has a desired activity, and the solution 1 contains the multilayer particle 10 and the hydrophobic solvent 20.

The hydrophobic solvent 20 is intended to be a liquid that is a low-polarity solvent and that is not mixed with, or hardly mixed with, water. The hydrophobic solvent 20 is preferably a liquid that has low cytotoxicity. The hydrophobic solvent 20 is preferably oil, examples of which include mineral oil, silicon oil, and fluorine-based oil.

In a case where the multilayer particle 10 is dispersed in the hydrophobic solvent 20, the detection layer 13 which is the outermost layer of the multilayer particle 10 can be formed as a layer of an aqueous solution. In a case where at least one of the detection layers 13 is a layer of an aqueous solution, for example, in the detection layer 13 of the aqueous solution, an efficiency with which both the substance secreted by the microorganism 12 and the detection body 14 are dispersed and an efficiency with which both the substance secreted by the microorganism 12 and the detection body 14 are prevented from being eluted from the multilayer particle 10 are greatly improved as compared with the case of a gel. This makes it possible to, for example, improve detection sensitivity of the detection body 14 and shorten a culture time after formation of the detection layer 13. By causing the detection layer 13 which is the outermost layer of the multilayer particle 10 to be a layer of an aqueous solution that is less viscous than a gel, it is easy to uniformly control the particle size of the multilayer particle 10.

The multilayer particle 10 may be contained in the solution 1 in an amount of 10² particles/mL to 10¹⁰ particles/mL, and the amount is preferably in a range of 10⁶ particles/mL to 10⁸ particles/mL in terms of promotion of an intercellular interaction and screening efficiency.

Note that the screening method in accordance with an embodiment of the present invention can be carried out also in a case where the multilayer particle 10 is dispersed in an aqueous solution rather than in the hydrophobic solvent 20. In this case, the detection layers 13 that are formed in the multilayer particle 10 are each a layer of a gel.

### [Steps of screening method]

The following description will discuss the screening method in accordance with an embodiment of the present invention which screening method is carried out by the multilayer particle 10 described earlier. The screening method includes a particle forming step (S1 to S3) and a selection step (S4 and S5) as illustrated in Fig. 2. Fig. 2 is a flowchart showing an example of the screening method in accordance with an embodiment of the present invention.

### (Particle forming step)

The particle forming step is a step of obtaining the multilayer particle 10 by forming, outside the core particle 11 formed from the solid culture medium containing the microorganism 12, at least one detection layer 13 that is composed of a gel or an aqueous solution and that contains the detection body 14 which detects the desired activity.

Specifically, the core particle 11 that contains the microorganism 12 is formed (S1). A common water/oil (W/O) emulsion formation method as disclosed in Patent Literature 1 can be used to form the core particle 11.

For example, by mixing the hydrophobic solvent 20 with an aqueous solution obtained by mixing a solid culture medium in a sol state before solidification, the microorganism 12, and a surfactant for an aqueous phase, and vigorously stirring a resulting mixture, a state in which particles in the aqueous phase are dispersed in the hydrophobic solvent 20 is formed. Gelling the particles in the aqueous phase by refrigeration or the like makes it possible to form the core particle 11.

The number (density) of microorganisms 12 in the foregoing aqueous solution is preferably adjusted so that the microorganism 12 consisting of one cell is included in each of the core particles 11. As a result of this, a colony in which a single microorganism has proliferated is formed in each of multilayer particles 10.

Next, culture of the microorganism 12 in the core particle 11 is preferably carried out for a prescribed period (S2). Carrying out the culture results in proliferation of the microorganism 12. This makes it possible to detect the desired activity with higher sensitivity. Even if the desired activity is an activity that is not expressed unless the microorganism 12 proliferates to form a colony, the desired activity can be detected without any problems.

The core particle 11 may be cultured in a state of being dispersed in the hydrophobic solvent 20 that was used to form the core particle 11, or may be cultured by replacing the solvent with the hydrophobic solvent 20 of another type or an aqueous solution. The solvent may be replaced as appropriate also in each of subsequent steps.

In a case where it is possible to estimate a type of the microorganism 12 that has the desired activity, a prescribed period for culture may be a period suitable for the type. The prescribed period may be determined as appropriate while the microorganism 12 in the core particle 11 is being observed, or may be empirically determined. A culture temperature may be similarly determined as appropriate. For example, the culture time may be 1 hour to 1,000 hours, and the culture temperature may be 4°C to 70°C.

Subsequently, the detection layer 13 that contains the detection body 14 is formed outside the core particle 11 to obtain the multilayer particle 10 (S3). A method in which the core particle 11 is used as a core to form a W/O emulsion can be used to form the detection layer 13.

For example, the core particle 11 that serves as an inner layer is mixed with a gel solution or aqueous solution that contains the detection body 14 and that is in a sol state before solidification. Thereafter, by forming a W/O emulsion by the foregoing method, the multilayer particle 10 in which the detection layer 13 is formed outside the core particle 11 is obtained. In this case, adjusting the number (density) of core particles 11 with respect to the volume of the gel solution or aqueous solution makes it possible to adjust the number of core particles 11 that are contained in one multilayer particle 10 in each W/O emulsion formed. For example, in a case where the multilayer particle 10 that includes a single core particle 11 is formed, the multilayer particle 10 need only be formed by adjusting the number (density) of core particles 11 so that zero or one core particle 11 is encapsulated in the detection layer 13.

Another layer other than the core particle 11 and the detection layer 13 may be further formed in the multilayer particle 10. In this case, a gel solution or aqueous solution containing a substance that is contained in the another layer is mixed with the core particle 11 or the multilayer particle 10 to form a W/O emulsion, so that the multilayer particle 10 in which the another layer is formed is obtained.

In a case where the detection layer 13 is formed as a layer of an aqueous solution, the layer of the aqueous solution is formed as the outermost layer of the multilayer particle 10 in the hydrophobic solvent 20. The detection layer 13 of the aqueous solution can be stably maintained as the outermost layer of the multilayer particle 10 in the hydrophobic solvent 20. The outermost layer of the multilayer particle 10 may be used as the another layer that is composed of an aqueous solution.

### (Selection step)

The selection step is a step of selecting, on the basis of a signal indicating a result of detection of the desired activity by the detection body 14, the multilayer particle 10 that contains the microorganism 12 that has the desired activity.

First, culture of the multilayer particle 10 that has been formed in the particle forming step is preferably carried out for a prescribed period (S4). The culture makes it possible to react a secreted substance, etc., of the microorganism 12 that is contained in the core particle 11 with the detection body 14 that is contained in the detection layer 13. Culture conditions such as the prescribed period and a culture temperature may be determined as appropriate in accordance with the desired activity and a type of the detection body 14.

The multilayer particle 10 may be cultured in a state in which the multilayer particle 10 that has been formed in the hydrophobic solvent 20 is dispersed as it is in the hydrophobic solvent 20. Alternatively, the multilayer particle 10 may be cultured by dispersing the multilayer particle 10 in another type of the hydrophobic solvent 20 or an aqueous solution.

Next, the multilayer particle 10 is selected on the basis of the signal of the detection body 14 (S5). Here, a case where the desired activity is antimicrobial activity and the detection body 14 is a cell into which a fluorescent protein has been introduced is described as an example. However, an aspect of the present invention is not limited to this. The screening method in accordance with an embodiment of the present invention is capable of handling, in accordance with the type of the detection body 14, various functions and activities of the microorganism 12, such as antimicrobial activity, a specific enzyme activity such as cellulase activity, or production activity of a useful substance.

Here, the multilayer particle 10 after culturing in step S4 is analyzed by a cell sorter, a droplet sorter, or the like. In accordance with specifications of the cell sorter or the droplet sorter, the multilayer particle 10 may be analyzed while being dispersed in the hydrophobic solvent 20, or may be analyzed by being dispersed in an aqueous solution. The multilayer particle 10 in which fluorescence emitted in the multilayer particle 10 by a fluorescent protein expressed by the detection body 14 has disappeared is selected as the multilayer particle 10 that contains the microorganism 12 that has the desired activity.

The selected multilayer particle 10 may be used for large-scale culture of the microorganism 12 that has the desired activity. For example, the microorganism 12 may be inoculated from the selected multilayer particle 10 into a culture medium which has a larger culture scale than that of the multilayer particle 10, and a cultured strain of the microorganism 12 that has the desired activity may be obtained. According to such a method, the microorganism 12 that has the desired activity can be artificially proliferated and subjected to usage suitable for the activity.

Further, by, for example, carrying out genome sequencing of the microorganism 12 encapsulated in the selected multilayer particle 10, the selected multilayer particle 10 can also be used for searching for a gene that expresses the desired activity. In a case where the gene can be estimated or identified, for example, even if artificial large-scale culture of the microorganism 12 that has the desired activity is difficult, the desired activity can be easily reproduced by introducing the gene into a cell that is relatively easy to culture.

According to the foregoing configuration, a large number of multilayer particles 10 can be used to detect the desired activity of the microorganism 12. This makes it possible to use a cell sorter or the like to screen, with an efficiency which far exceeds that of the prior art, a microorganism that has the desired activity.

In particular, in a screening system using a gel microdroplet (GMD) technique, a cell, a substrate, a reagent, or the like that is necessary for detection of the desired activity can be added at a desired timing, that is, even after the step of culturing the microorganism 12. This makes it possible to evaluate various activities that have not conventionally been detectable by the screening system using the GMD technique.

For example, in a case where a cell itself is used as the detection body 14 that detects antimicrobial activity, or in a case where a substrate serving as the detection body 14 that detects, for example, cellulase activity is a polymer, it is difficult in many cases to add the detection body 14 from outside a GMD. According to the screening method in accordance with an embodiment of the present invention, after a microorganism is cultured and proliferated, a layer of a gel or an aqueous solution containing the detection body 14 can be formed to create the multilayer particle 10, and a large number of multilayer particles 10 can be used to screen the desired activity with high throughput.

Aspects of the present invention can also be expressed as follows:
A screening method in accordance with Aspect 1 of the present invention is a screening method for searching for a desired microorganism that is included in a group of microorganisms and that has a desired activity, the screening method including: a particle forming step of obtaining a multilayer particle by forming, outside a core particle formed from a solid culture medium containing the microorganisms, at least one detection layer that is composed of a gel or an aqueous solution and that contains a detection body which detects the desired activity; and a selection step of selecting, on the basis of a signal indicating a result of detection of the desired activity by the detection body, the multilayer particle that contains the desired microorganism, in the particle forming step, in a case where the at least one detection layer is formed as a layer that is composed of the aqueous solution, the layer being formed as an outermost layer of the multilayer particle in a hydrophobic solvent.

In Aspect 2 of the present invention, a screening method may be configured such that, in Aspect 1, the detection body is a cell different from the desired microorganism.

In Aspect 3 of the present invention, a screening method may be configured such that, in Aspect 2, the detection body is the cell into which a fluorescent substance or a light-emitting substance has been introduced.

In Aspect 4 of the present invention, a screening method may be configured such that, in Aspect 3, the desired activity is antimicrobial activity, and in the selection step, a reduction in fluorescence emitted by the fluorescent substance or in light emission by the light-emitting substance, the reduction being caused by a reduction in number of cells, each of which is the detection body, is used as the signal which indicates that the detection body has detected the desired activity.

In Aspect 5 of the present invention, a screening method may be configured such that, in Aspect 1, the at least one detection layer which is the outermost layer of the multilayer particle is formed as the layer that is composed of the aqueous solution, and the detection body which is contained in the at least one detection layer which is the outermost layer of the multilayer particle and which is formed as the layer that is composed of the aqueous solution is a water-soluble compound.

In Aspect 6 of the present invention, a screening method may be configured such that, in Aspect 1 or 5, the desired activity is cellulase activity, the detection body is a fluorescent substance that emits fluorescence due to an influence of the cellulase activity or a light-emitting substance that emits light due to the influence of the cellulase activity, and in the selection step, an increase in fluorescence emitted by the fluorescent substance or in light emission by the light-emitting substance is used as the signal which indicates that the detection body has detected the desired activity.

A solution in accordance with Aspect 7 of the present invention is a solution which is used to search for a desired microorganism that is included in a group of microorganisms and that has a desired activity and which contains a multilayer particle and a hydrophobic solvent, the multilayer particle including: a core particle formed from a solid culture medium containing the microorganisms; and at least one detection layer that is formed outside the core particle, that is composed of a gel or an aqueous solution, and that contains a detection body which detects the desired activity, wherein in a case where the multilayer particle includes the at least one detection layer that is composed of the aqueous solution, the at least one detection layer that is composed of the aqueous solution is an outermost layer of the multilayer particle.

### Examples

The following description will discuss an example of the present invention.

A model microorganism was used to confirm whether using the multilayer particle 10 that is a GMD including a plurality of layers makes it possible to rapidly detect and select, from among a large number of microorganisms 12, the microorganism 12 that has the desired activity.

In the present example, such confirmation was carried out assuming that the desired activity is antimicrobial activity. Lactic acid bacteria (microorganism 12) that produces an antibacterial substance was formed in the core particle 11 which serves as an inner layer, and the multilayer particle 10 that contains fluorescent protein-expressing Escherichia coli was formed, as the detection body 14 for detecting the antibacterial substance, in the detection layer 13 which serves as a second layer. A cell sorter was used to select the multilayer particle 10 in which fluorescence of Escherichia coli had decreased due to the antibacterial substance produced by the lactic acid bacteria, and the multilayer particle 10 that had been selected by carrying out culturing was checked. The present example is described below in detail.

### [1. Screening of microorganism having antimicrobial activity]

### <1-1. Formation of core particle>

First, lactic acid bacteria (Lactobacillus paracasei subsp. paracasei NBRC 15889 strain) for being encapsulated in the core particle 11 were cultured. The present lactic acid bacteria strain forms an inhibition zone in a susceptibility test using Escherichia coli (Escherichia coli BL21 strain).

Lactic acid bacteria were inoculated into a de Man-Rogosa-Sharpe (MRS) agar medium containing 1% calcium carbonate, and were subjected to pre-culture in anaerobic conditions at 28°C for 24 hours. Around a grown lactic acid bacteria colony, calcium carbonate in a culture medium was dissolved due to production of lactic acid, and a transparent halo was formed. On the day before formation of the core particle 11, a colony having a halo formed therein was scraped off with a microloop, inoculated into 5 mL of an MRS liquid culture medium, and subjected to static culture at 28°C for 16 hours.

Bacterial cells were collected by centrifugation and suspended in PBS. A portion of a bacterial cell suspension was subjected to nucleic acid staining in the presence of 1×SYBR GreenI, a fluorescence microscope was used to count the number of cells, and a cell concentration of the bacterial cell suspension was calculated.

SeaPlaque (registered trademark) agarose was used as a hydrogel for forming the core particle 11. 500 µL of 1.5 w/v% agarose was heated at 70°C and melted. After being melted, the agarose was kept warm at 40°C. To the agarose that was kept warm, 100 µL of a PBS cell suspension containing 25 µL of a Poloxamer 188 solution, 50 µL of an MRS medium at a 10-fold concentration, and 5.0×10⁸ lactic acid bacteria cells was added, and a resulting mixture was well mixed with pipetting.

A mineral oil containing a surfactant Span 80 at a concentration of 1% was kept warm at 40°C. To the mineral oil, an agarose mixture was added, and a resulting mixture was subjected to inversion mixing and stirred with a CellSys 100 microdrop maker at 1,300 rpm for 2 minutes to form a micelle serving as the core particle 11. Thereafter, the rotation speed was reduced to 150 rpm, and a gel was solidified by carrying out stirring for 7 minutes while carrying out cooling with ice water.

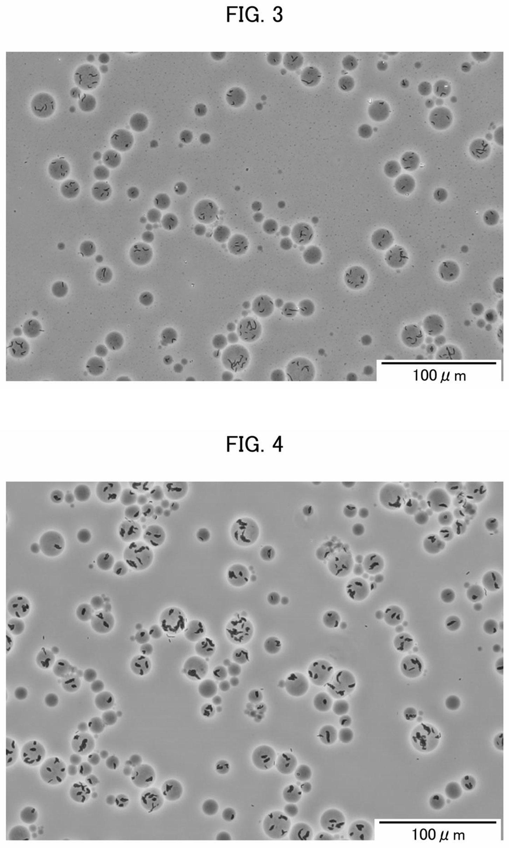

Some of core particles 11 formed were washed with PBS to remove the oil, and observation was carried out under a microscope. As a result, as illustrated in Fig. 3, core particles 11 having diameters of 5 µm to 30 µm were formed, and zero to two or more lactic acid bacteria cells were contained per core particle 11.

A mixture of the core particles 11 thus obtained and the oil was sealed, and culture was carried out at room temperature for 2 days while stirring was being carried out with a rotator at 10 rpm. After culturing, the oil and the core particles 11 were separated by centrifugation, and the core particles 11 were collected and washed with an MRS liquid culture medium to remove the oil.

As a result of microscopic observation of the collected core particles 11, proliferation of lactic acid bacteria in the core particles 11 was observed, as illustrated in Fig. 4.

### <1-2. Formation of multilayer particle>

As the detection body 14 to be added to the detection layer 13, pRSET-EmGFP Bacterial Expression Vector (Invitrogen) was introduced, and Escherichia coli BL21 strain expressing a green fluorescent protein (hereafter referred to as Escherichia coli pRSET-EmGFP) was prepared. An excitation wavelength/a fluorescence wavelength of green fluorescence of the present Escherichia coli is 487 nm/509 nm.

As pre-culture, Escherichia coli pRSET-EmGFP was inoculated onto a Luria-Bertani (LB) agar medium containing ampicillin at a concentration of 100 µg/mL, and cultured for 16 hours to form a colony. On the day before formation of the multilayer particle 10, a single colony was inoculated into 5 mL of an LB liquid culture medium containing ampicillin at the same concentration, and cultured at 37°C for 16 hours while being shaken at 112 rpm.

Escherichia coli pRSET-EmGFP was collected by centrifugation and suspended in PBS. A portion of a bacterial cell suspension was subjected to nucleic acid staining in the presence of 1×SYBR (registered trademark) Green I, a fluorescence microscope was used to count the number of cells, and a cell concentration of the bacterial cell suspension was calculated.

SeaPlaque agarose was used as a hydrogel of the detection layer 13 as in the case of that of the core particle 11. After melting, to 1 mL of 1.5 w/v% agarose that was kept warm at 40°C, the core particle 11 containing the aforementioned lactic acid bacteria and 100 µ of a PBS cell suspension containing 50 µL of a Poloxamer 188 solution, 100 µL of an MRS medium at a 10-fold concentration, and 4.0×10⁹ Escherichia coli pRSET-EmGFP cells were added in a volume of 100 µL, and a resulting mixture was well mixed with pipetting.

A mineral oil containing 1% Span 80 was kept warm at 40°C. To the mineral oil, the aforementioned agarose mixture was added, and a resulting mixture was subjected to inversion mixing and stirred with a CellSys 100 microdrop maker at 1,300 rpm for 2 minutes to create a micelle encapsulating the core particle 11 containing the lactic acid bacteria. Thereafter, the rotation speed was reduced to 150 rpm, and a gel was solidified by carrying out stirring for 7 minutes while carrying out cooling with ice water.

In order to use a cell sorter to detect fluorescence disappearance in Escherichia coli pRSET-EmGFP due to lactic acid bacteria, as a positive control, a multilayer particle not containing Escherichia coli pRSET-EmGFP in an outer layer of the core particle 11 was created. Instead of a suspension of Escherichia coli pRSET-EmGFP, 100 µL of PBS was added to create the multilayer particle by a method similar to that in the case of the multilayer particle 10.

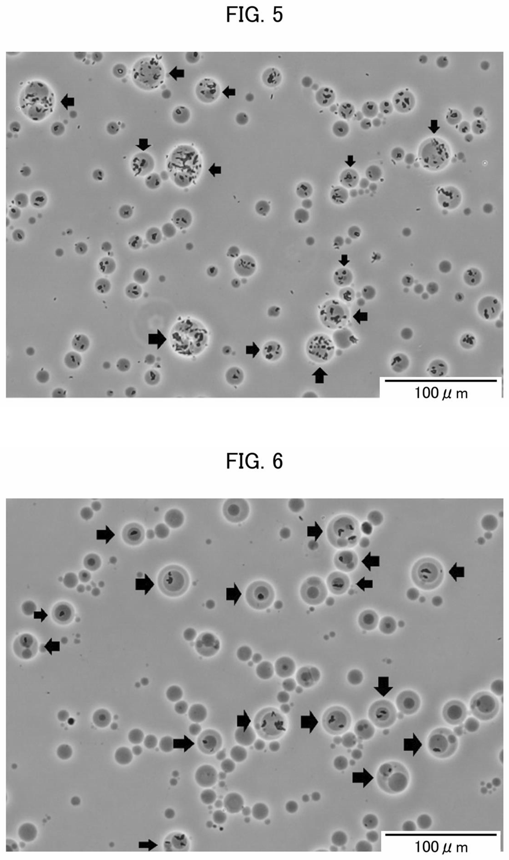

Fig. 5 shows an image obtained by washing some of created multilayer particles 10 with PBS to remove the oil and carrying out observation under a microscope. Approximately one tenth of the multilayer particles 10 and single-layer particles of Escherichia coli pRSET-EmGFP were observed. In Fig. 5, the multilayer particles 10 are shown by arrows. The multilayer particles 10 have diameters of 20 µm to 40 µm, and almost all the multilayer particles 10 contained Escherichia coli pRSET-EmGFP serving as the detection body 14. Fig. 6 is a microscopic image of multilayer particles serving as a positive control and each not containing Escherichia coli pRSET-EmGFP in an outer layer thereof. The multilayer particles each containing lactic acid bacteria in the core particle 11 are shown by arrows.

A mixture of the aforementioned multilayer particles 10 and the oil was sealed, and culture was carried out at room temperature for 6 days while stirring was being carried out with a rotator at 10 rpm. After culturing, the oil and the multilayer particles 10 were separated by centrifugation, and the multilayer particles 10 were collected and washed with PBS to remove the oil.

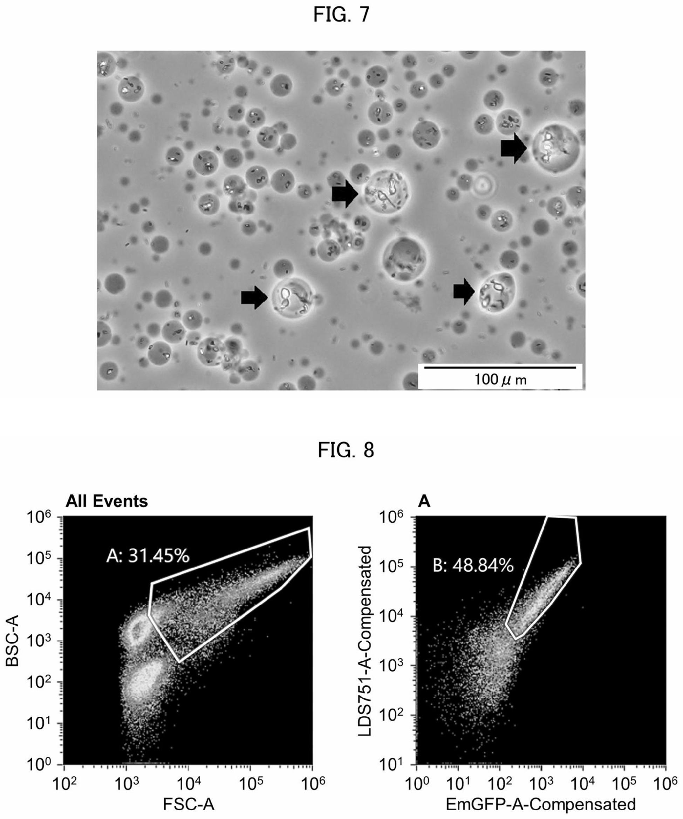

Fig. 7 shows a microscopic image of the multilayer particles 10 after culturing each containing Escherichia coli pRSET-EmGFP in an outer layer thereof. As shown by arrows in Fig. 7, colonies of lactic acid bacteria that had developed inside the multilayer particles 10 were observed.

### <1-3. Selection of multilayer particle>

The multilayer particles 10 that had been washed with PBS was allowed to pass through a nylon membrane filter with a pore size of 20 µm so that a flow path of the cell sorter was not blocked.

The multilayer particles 10 were stained under a final concentration of 10 µM with use of LDS 751 (excitation/fluorescence wavelength = 543 nm/712 nm) that is a nucleic acid staining reagent which has cell membrane permeability and which is for carrying out whole bacterial staining.

Cell Sorter SH800S manufactured by Sony Group Corporation was used as the cell sorter, and a sorting chip with an orifice diameter of 130 µm was used. A 488-nm laser was used as a fluorescence excitation light source, and forward-scattered light (FSC) and backscattered light (BSC) were used to detect the multilayer particles 10. A sensor having an FL1 (500 nm to 550 nm) optical filter and a sensor having an FL5 (690 nm to 750 nm) optical filter were used to detect green fluorescence of EmGFP and red fluorescence derived from LDS 751, respectively.

First, multilayer particles serving as a positive control and each not containing Escherichia coli pRSET-EmGFP in an outer layer thereof were subjected to measurement with the cell sorter. First, BSC and FSC were set on the vertical and horizontal axes of a dot plot to display respective measurement values of the multilayer particles, and an area A was set in a region in which a group of the multilayer particles or the core particles 11 is distributed. Next, for the multilayer particles or the core particles 11 distributed in the area A, fluorescence intensities thereof were displayed in a dot plot in which FL5 (LDS 751 fluorescence intensity) and FL1 (EmGFP fluorescence intensity) were set on the vertical and horizontal axes. Multilayer particles with a high red fluorescence intensity of LDS 751 were set as an acquisition target, and an area B was set in a region in which a group of the multilayer particles is distributed.

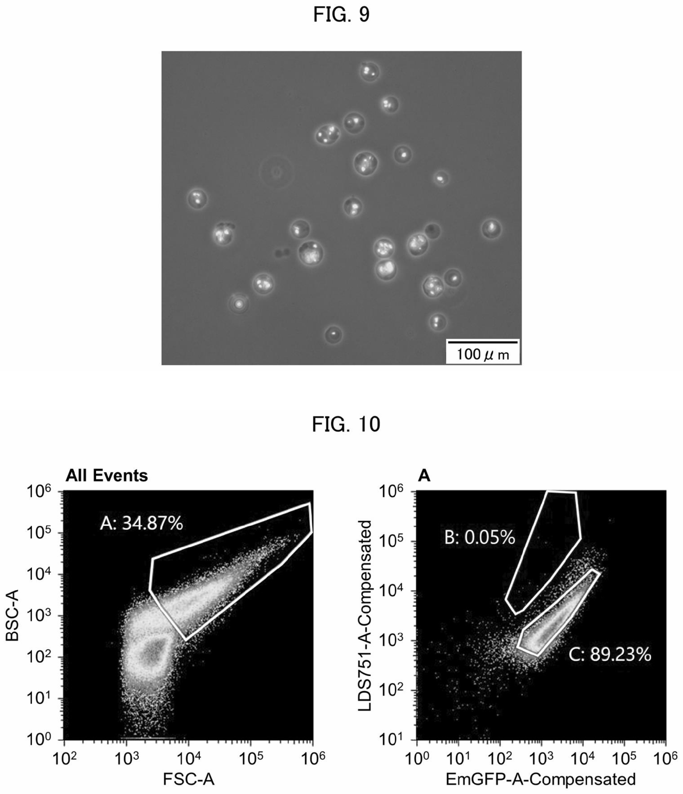

Fig. 8 shows a dot plot of the multilayer particles subjected to measurement or the core particles 11 and a set area, and Fig. 9 shows an image of particles sorted from the area B. In Fig. 9, a part that emits fluorescence (white part inside a particle) is a colony of lactic acid bacteria.

Next, the multilayer particle 10 including the detection layer 13 containing Escherichia coli pRSET-EmGFP was subjected to measurement under a condition similar to the aforementioned condition. An area C was newly set on the dot plot of FL1 and FL5 for a group of multilayer particles 10 each of which does not contain lactic acid bacteria or in which proliferation of lactic acid bacteria is less likely to be observed. Fig. 10 shows a cell sorter dot plot in the present condition.

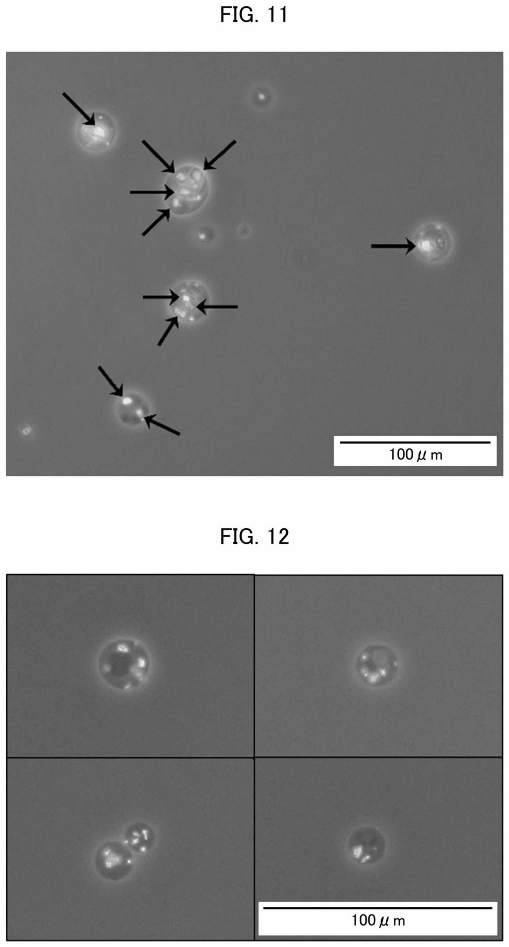

The multilayer particles 10 that were acquisition targets and that were distributed in the area B and the area C were sorted and observed under a fluorescence microscope. Fig. 11 illustrates the multilayer particles 10 that have been sorted from the area B, and Fig. 12 illustrates the multilayer particles 10 that have been sorted from the area C.

In Fig. 11, a white part shown by an arrow indicates a colony of lactic acid bacteria inside the multilayer particle 10, and fluorescence derived from Escherichia coli pRSET-EmGFP is not detected. In Fig. 12, a white part inside the multilayer particle 10 indicates Escherichia coli pRSET-EmGFP, and fluorescence derived from a colony of lactic acid bacteria is not detected. Thus, in each of the multilayer particles 10 sorted from the area B, a colony of lactic acid bacteria in the core particle 11 and fluorescence disappearance of Escherichia coli pRSET-EmGFP in the detection layer 13 were observed. In the multilayer particles 10 sorted from the area C, Escherichia coli pRSET-EmGFP that emits green fluorescence was observed.

The multilayer particles 10 distributed in each of the area B and the area C were sorted, inoculated on a microplate petri dish containing an MRS agar medium containing 1% calcium carbonate, and cultured in anaerobic conditions at 28°C for 6 days. A colony of lactic acid bacteria was determined in accordance with the presence or absence of a halo around the colony.

87% of lactic acid bacteria colonies appeared from the multilayer particles 10 in the area B, whereas only 4% of lactic acid bacteria colonies appeared from the multilayer particles 10 in the area C. This result shows that the screening method in accordance with an embodiment of the present invention has made it possible to accurately select the multilayer particle 10 that contains lactic acid bacteria. The screening method is also considered to have made it possible to select, from among a group of lactic acid bacteria, the multilayer particle 10 that has lactic acid bacteria having a high ability to produce an antibacterial substance.

The present example has confirmed that using the multilayer particle 10 makes it possible to rapidly determine, from among a large number of microorganisms, a microorganism which has a desired activity, and acquire the microorganism.

### [2. Screening of microorganism having cellulase activity]

### <2-1. Formation of core particle>

First, cellulase-producing bacteria (Cellulomonas sp. 16064 strain) for being encapsulated in the core particle 11 were cultured.

The cellulase-producing bacteria were inoculated into an agar medium (2 g/L of hipolypepton, 0.4 g/L of yeast extract, and 0.2 g/L of MgSO₄·7H₂O) and subjected to pre-culture for 24 hours. On the day before formation of the core particle 11, a colony of the cellulase-producing bacteria was scraped off with a microloop, inoculated into 5 mL of a 1% carboxymethyl cellulose (CMC)-containing liquid culture medium (0.5 g/L of KH₂PO₄, 0.1 g/L of CaCl₂, 6.0 g/L of NaCl, 0.5 g/L of K₂HPO₄, 0.1 g/L of NaNO₃, 1.0 g/L of (NH₄)₂SO₄, 0.1 g/L of MgSO₄, and 1.0 g/L of yeast extract), and cultured at 28°C for 24 hours while being shaken at 200 rpm.

Bacterial cells were collected by centrifugation and suspended in PBS. A portion of a bacterial cell suspension was subjected to nucleic acid staining in the presence of 1×SYBR GreenI, a fluorescence microscope was used to count the number of cells, and a cell concentration of the bacterial cell suspension was calculated.

SeaPlaque (registered trademark) agarose was used as a hydrogel for forming the core particle 11. 500 µL of 1.5 w/v% agarose was heated at 70°C and melted. After being melted, the agarose was kept warm at 40°C. To the agarose that was kept warm, 50 µL of the liquid culture medium at a 5-fold concentration not containing CMC and 100 µL of a PBS cell suspension containing 1.1×10⁸ cellulase-producing bacteria cells were added, and a resulting mixture was well mixed with pipetting.

A mineral oil containing a surfactant Span 80 at a concentration of 1% was kept warm at 40°C. To the mineral oil, an agarose mixture was added, and a resulting mixture was subjected to inversion mixing and stirred with a CellSys 100 micro drop maker at 1,300 rpm for 2 minutes to form a micelle serving as the core particle 11. Thereafter, the rotation speed was reduced to 150 rpm, and a gel was solidified by carrying out stirring for 7 minutes while carrying out cooling with ice water.

Some of core particles 11 formed were washed with the liquid culture medium containing 1% CMC to remove the oil. A mixture of the core particles 11 thus obtained and the oil was sealed, and culture was carried out at room temperature for 6 days while stirring was being carried out with a rotator at 175 rpm. After culturing, the oil and the core particles 11 were separated by centrifugation, and the core particles 11 were collected and washed with PBS to remove the oil.

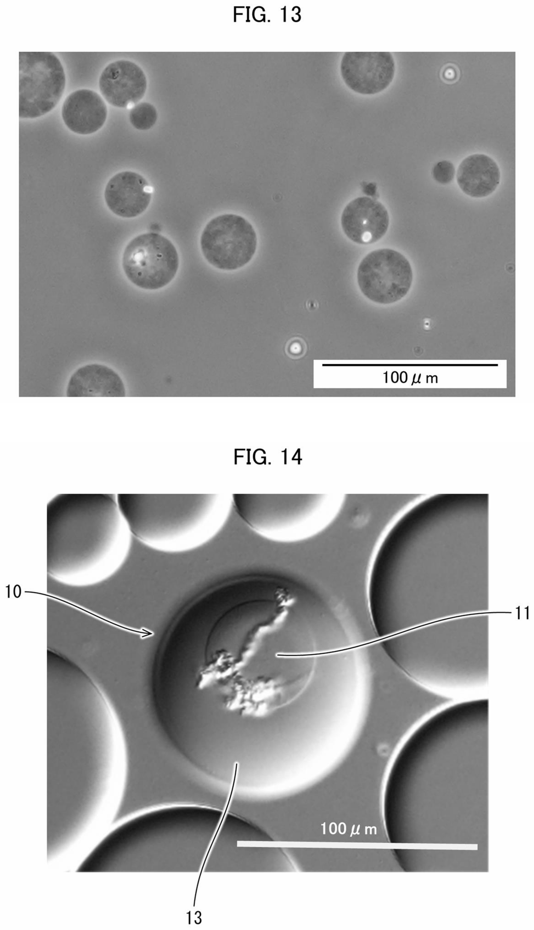

As a result of microscopic observation of the collected core particles 11, proliferated cellulase-producing bacteria were observed in the core particles 11 having diameters of 5 µm to 30 µm, as illustrated in Fig. 13.

### <2-2. Formation of multilayer particle>

A GMD in a water-in-oil droplet (WODL) which is an aspect of the multilayer particle 10 and in which the detection layer 13 is composed of an aqueous solution was created. As the detection body 14 to be added to the detection layer 13, fluorescein di-beta-D-cellobioside (AAT Bioquest) that is a cellulase fluorescent substrate was used.

To 1 mL of the liquid culture medium (note, however, that a final concentration of the CMC was set to 0.1%) serving as the detection layer 13, 6.1×10³ core particles 11 containing the cellulase-producing bacteria and fluorescein di-beta-D-cellobioside were added, and a resulting mixture was well mixed with pipetting. Fluorescein di-beta-D-cellobioside was added so as to achieve a final concentration of 40 µM.

A resulting mixed solution and a fluorine oil containing 008-FluoroSurfactant-5wtH (On-chip Biotechnologies) at a concentration of 2 w/w% were supplied to an On-chip (registered trademark) droplet generator, and a GMD in a WODL in which the detection layer 13 had been formed from the liquid culture medium that is an aqueous solution was obtained in the fluorine oil. As a result of microscopic observation, the GMD in the WODL that is the multilayer particle 10 containing a cellulase-producing bacteria-containing core particle 11 and the detection layer 13 composed of the aqueous solution was observed as illustrated in Fig. 14.

### <2-3. Selection of multilayer particle with use of model system>

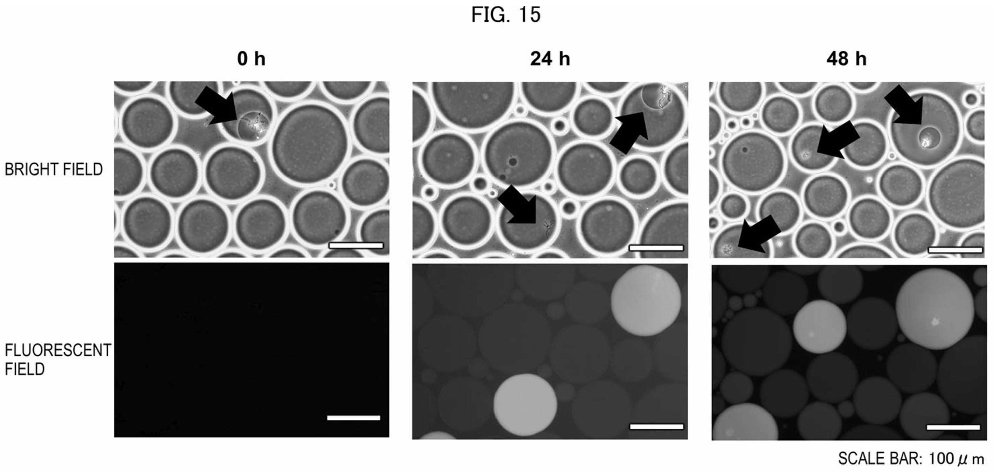

The GMD in the WODL thus created was allowed to stand still in a dark place and cultured at room temperature for 24 hours or 48 hours. Fig. 15 shows a result of microscopic observation of the GMD in the WODL after culturing for 24 hours or 48 hours in a bright field and a fluorescent field. In observation in the fluorescent field, blue excitation light at 460 nm to 495 nm was used for a fluorescence excitation light source, and an optical filter that transmits light at 510 nm or more was used to detect green fluorescence.

The GMD in the WODL containing cellulase-producing bacteria was observed as shown by arrows in microscopic images in the bright field in Fig. 15. Further, in microscopic images in the fluorescent field, green fluorescence was detected both after culturing for 24 hours and after culturing for 48 hours. This shows that, in the GMD in the WODL which includes a cellulase-producing bacteria-containing core particle 11, fluorescein di-beta-D-cellobioside of the detection layer 13 has been cleaved into fluorescein by cellulase produced by the cellulase-producing bacteria, and the fluorescein has emitted green fluorescence.

This result shows that the screening method in accordance with an embodiment of the present invention has made it possible to accurately select the GMD in the WODL that is the multilayer particle 10 containing cellulase-producing bacteria. Further, the result shows that the multilayer particle 10 in which the detection layer 13 is composed of an aqueous solution is also suitably available for the screening method in accordance with an embodiment of the present invention.

The present example has confirmed that using the multilayer particle 10 makes it possible to rapidly determine, from among a large number of microorganisms, a microorganism which has a desired activity, and acquire the microorganism.

### <2-4. Selection of multilayer particle containing microorganism in environment>

Screening of cellulase-producing bacteria was attempted from soil bacteria. Here, a GMD in a WODL was created in accordance with the methods described in <2-1. Formation of core particle> and <2-2. Formation of multilayer particle>, except that soil bacteria collected from forest soil on the premises of Hiroshima University were used as the microorganism 12 to be encapsulated in the core particle 11.

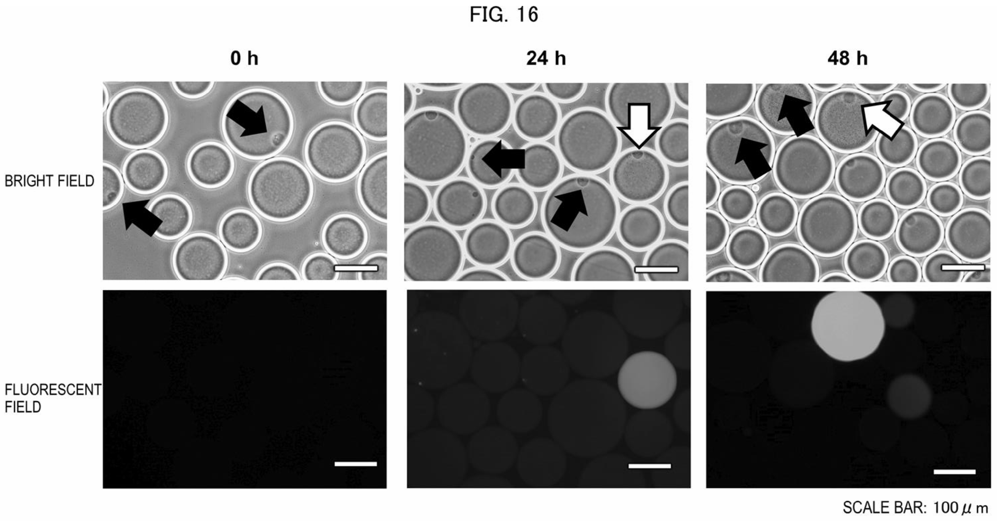

The GMD in the WODL thus created was allowed to stand still in a dark place and cultured at room temperature for 24 hours or 48 hours. Fig. 16 shows a result of microscopic observation of the GMD in the WODL after culturing for 24 hours or 48 hours in a bright field and a fluorescent field. An observation condition in the fluorescent field was the same condition as that in <2-3. Selection of multilayer particle with use of model system>.

The GMD in the WODL containing the microorganism 12 was observed as shown by solid black arrows and outlined white arrows in microscopic images in the bright field in Fig. 16. Further, in microscopic images in the fluorescent field, the GMD in the WODL that emits green fluorescence was detected both after culturing for 24 hours and after culturing for 48 hours. The GMD in the WODL that emits green fluorescence is shown by the outlined white arrows in the microscopic images in the bright field.

This is a result showing that it was possible to clearly detect, from among GMDs in WODLs each containing diverse soil bacteria, the GMD in the WODL which contains cellulase-producing bacteria having cellulase activity.

The present example has suggested that using the multilayer particle 10 makes it possible to rapidly determine, from among, for example, a large number of microorganisms present in the environment, a microorganism which has a desired activity, and acquire the microorganism.

### [Additional remarks]

The present invention is not limited to the embodiments or examples, but can be altered by a skilled person in the art within the scope of the claims. The present invention also encompasses, in its technical scope, any embodiment derived by combining technical means disclosed in differing embodiments or examples.

### Industrial Applicability

An embodiment of the present invention can be used to screen microorganisms having a desired activity useful for, for example, drug development.

### Reference Signs List

- 1: Solution
- 10: Multilayer particle
- 11: Core particle
- 12: Microorganism
- 13: Detection layer
- 14: Detection body
- 20: Hydrophobic solvent

## Claims

1. A screening method for searching for a desired microorganism that is included in a group of microorganisms and that has a desired activity, the screening method comprising:
a particle forming step of obtaining a multilayer particle by forming, outside a core particle formed from a solid culture medium containing the microorganisms, at least one detection layer that is composed of a gel or an aqueous solution and that contains a detection body which detects the desired activity; and
a selection step of selecting, on the basis of a signal indicating a result of detection of the desired activity by the detection body, the multilayer particle that contains the desired microorganism,
in the particle forming step, in a case where the at least one detection layer is formed as a layer that is composed of the aqueous solution, the layer being formed as an outermost layer of the multilayer particle in a hydrophobic solvent.

2. The screening method as set forth in claim 1, wherein the detection body is a cell different from the desired microorganism.

3. The screening method as set forth in claim 2, wherein the detection body is the cell into which a fluorescent substance or a light-emitting substance has been introduced.

4. The screening method as set forth in claim 3, wherein
the desired activity is antimicrobial activity, and
in the selection step, a reduction in fluorescence emitted by the fluorescent substance or in light emission by the light-emitting substance, the reduction being caused by a reduction in number of cells, each of which is the detection body, is used as the signal which indicates that the detection body has detected the desired activity.

5. The screening method as set forth in claim 1, wherein
the at least one detection layer which is the outermost layer of the multilayer particle is formed as the layer that is composed of the aqueous solution, and
the detection body which is contained in the at least one detection layer which is the outermost layer of the multilayer particle and which is formed as the layer that is composed of the aqueous solution is a water-soluble compound.

6. The screening method as set forth in claim 1 or 5, wherein
the desired activity is cellulase activity,
the detection body is a fluorescent substance that emits fluorescence due to an influence of the cellulase activity or a light-emitting substance that emits light due to the influence of the cellulase activity, and
in the selection step, an increase in fluorescence emitted by the fluorescent substance or in light emission by the light-emitting substance is used as the signal which indicates that the detection body has detected the desired activity.

7. A solution which is used to search for a desired microorganism that is included in a group of microorganisms and that has a desired activity and which contains a multilayer particle and a hydrophobic solvent,
the multilayer particle including:
a core particle formed from a solid culture medium containing the microorganisms; and
at least one detection layer that is formed outside the core particle, that is composed of a gel or an aqueous solution, and that contains a detection body which detects the desired activity, wherein
in a case where the multilayer particle includes the at least one detection layer that is composed of the aqueous solution, the at least one detection layer that is composed of the aqueous solution is an outermost layer of the multilayer particle.
